# EUROPEAN PATENT APPLICATION

(11) **EP 4 825 306 A1**
(43) Date of publication of application: **21.10.2026**
(21) Application number: 24902927.3
(22) Date of filing: 12.12.2024
(51) Int. Cl.: A61B 5/00, A61C 19/04

(54) **CONTROL METHOD AND APPARATUS FOR THREE-DIMENSIONAL SCANNING DEVICE, TERMINAL DEVICE, AND SYSTEM**

(30) Priority: 14.12.2023 CN 202311729669
(71) Applicant: Shining 3D Tech Co., Ltd., Hangzhou, Zhejiang 311258 (CN)
(72) Inventor: SUN, Bo, Hangzhou, Zhejiang 311258 (CN); ZHAO, Xiaobo, Hangzhou, Zhejiang 311258 (CN); CHEN, Xiaojun, Hangzhou, Zhejiang 311258 (CN); ZHANG, Huiquan, Hangzhou, Zhejiang 311258 (CN); MA, Chao, Hangzhou, Zhejiang 311258 (CN); LI, Chen, Hangzhou, Zhejiang 311258 (CN)
(74) Representative: Dehns Germany Partnerschaft mbB
(86) International application number: PCT/CN2024/138950
(87) International publication number: WO 2025/124510

(57) **Abstract**

The present invention provides a control method and apparatus for a three-dimensional scanning device, a terminal device, and a system. The method includes: acquiring a target image collected by the three-dimensional scanning device; determining, a current scanning environment type of the three-dimensional scanning device based on the target image; switching a current operating mode of the three-dimensional scanning device to a target operating mode matching the scanning environment type, or prompting the user to switch the current operating mode of the three-dimensional scanning device to the target operating mode matching the scanning environment type, wherein the three-dimensional scanning device is configured with at least two operating modes, each of the operating modes corresponds to one scanning environment type, when the three-dimensional scanning device is in the different operating modes, in a process of performing three-dimensional reconstruction on a target object based on an image collected by the three-dimensional scanning device, a processing method for processing the image is different, and/or, in a process of collecting the image using the three-dimensional scanning device, an operational state of each device in the three-dimensional scanning device is different.

## Description

### Cross-Reference to Related Application

The present invention claims priority to Chinese Patent Application No. 202311729669. X, filed to the China National Intellectual Property Administration on December 14, 2023 and entitled "Control Method and Apparatus for Three-Dimensional Scanning Device, Terminal Device, and System", the invention of which is hereby incorporated by reference in its entirety.

### Technical Field

The present invention relates to the field of three-dimensional scanning technology, and in particular, to a control method and apparatus for a three-dimensional scanning device, a terminal device, and a system.

### Background

With the development of three-dimensional scanning technology, the three-dimensional scanning technology is widely applied in various fields such as medical treatment, industry, and archaeology to perform three-dimensional reconstruction of a target object. When a user uses a three-dimensional scanning device to scan the target object, in order to obtain an ideal reconstruction effect, generally, for different types of scanning environments, there are differences in processing methods of images when three-dimensional reconstruction is subsequently performed using the images collected by the three-dimensional device. At present, when switching an operating mode of the three-dimensional scanning device, it is mainly performed by manual control of the user. The user may forget to switch the operating mode, resulting in an inappropriate processing method for the image in a current scanning environment. This leads to poor three-dimensional reconstruction effects, and requiring re-performing three-dimensional reconstruction or re-scanning.

### Summary

The present invention provides a control method and apparatus for a three-dimensional scanning device, a terminal device, and a system.

According to a first aspect of the embodiments of the present invention, a control method for a three-dimensional scanning device is provided. The method including:
acquiring a target image collected by the three-dimensional scanning device;
determining a current scanning environment type of the three-dimensional scanning device based on the target image;
switching a current operating mode of the three-dimensional scanning device to a target operating mode matching the scanning environment type, or prompting a user to switch the current operating mode of the three-dimensional scanning device to the target operating mode matching the scanning environment type;
wherein the three-dimensional scanning device is configured with at least two operating modes, each of the operating modes corresponds to one scanning environment type, when the three-dimensional scanning device is in the different operating modes, in a process of performing three-dimensional reconstruction on a target object based on an image collected by the three-dimensional scanning device, a processing method for processing the image is different, and/or, an operational state of each component in the three-dimensional scanning device during a process of collecting the image using the three-dimensional scanning device is different..

According to a second aspect of the embodiments of the present invention, a control apparatus for a three-dimensional scanning device is provided. The apparatus including:
an acquisition module, configured to acquire a target image collected by the three-dimensional scanning device;
a scanning environment type determination module, configured to determine a current scanning environment type of the three-dimensional scanning device based on the target image;
a processing module, configured to switch a current operating mode of the three-dimensional scanning device to a target operating mode matching the scanning environment type, or prompting a user to switch the current operating mode of the three-dimensional scanning device to the target operating mode matching the scanning environment type; wherein
the three-dimensional scanning device is configured with at least two operating modes, each of the operating modes corresponds to one scanning environment type, when the three-dimensional scanning device is in the different operating modes, in a process of performing three-dimensional reconstruction on a target object based on an image collected by the three-dimensional scanning device, a processing method for processing the image is different, and/or, an operational state of each component in the three-dimensional scanning device during a process of collecting the image using the three-dimensional scanning device is different.

According to a third aspect of the embodiments of the present invention, a terminal device is provided. The terminal device includes a processor, a memory, and computer instructions stored in the memory and executable by the processor. The processor, when executes the computer instructions, implements the control method for a three-dimensional scanning device mentioned in the above first aspect.

According to a fourth aspect of the embodiments of the present invention, a control system for a three-dimensional scanning device is provided. The control system includes a terminal device mentioned in the above third aspect, and the three-dimensional scanning device in communication connection with the terminal device. The three-dimensional scanning device is set to collect an image of an area to be scanned and send the image to the terminal device.

According to a fifth aspect of the embodiments of the present invention, a computer-readable storage medium is provided. Computer instructions are stored on the storage medium, and the computer instructions, when executed, implement the control method for a three-dimensional scanning device mentioned in the above first aspect.

In the embodiments of the present invention, a current scanning environment type of a three-dimensional scanning device can be automatically determined based on an image collected by the three-dimensional scanning device, and then an operating mode of the three-dimensional scanning device is automatically switched to a target operating mode adapted to the scanning environment type, or the user is prompted to switch the operating mode of the three-dimensional scanning device to the target operating mode adapted to the scanning environment type, thereby avoiding the issue that the user forgets to set the operating mode, resulting in a poor effect of a finally reconstructed three-dimensional model, and requiring re-performing three-dimensional reconstruction or re-scanning.

It should be understood that the above general description and the following detailed description are merely exemplary and explanatory, and do not limit the present invention.

### Brief Description of the Drawings

The accompanying drawings, which are incorporated in and constitute a part of the specification, illustrate embodiments consistent with the present invention and, together with the description, are used to explain the technical solutions of the present invention.
Fig. 1 is a schematic diagram of an application scenario according to an embodiment of the present invention.
Fig. 2 is a schematic diagram of configuring multiple operating modes for a three-dimensional scanning device according to an embodiment of the present invention.
Fig. 3 is a flowchart of a control method for a three-dimensional scanning device according to an embodiment of the present invention.
Fig. 4 is a schematic diagram of differences in processing methods between an intraoral operating mode and an extraoral operating mode according to an embodiment of the present invention.
Fig. 5 is a schematic diagram of obtaining a detection result of a scanning environment type based on a target image according to an embodiment of the present invention.
Fig. 6 is a schematic diagram of a logical structure of a control apparatus for a three-dimensional scanning device according to an embodiment of the present invention.
Fig. 7 is a schematic diagram of a logical structure of a terminal device according to an embodiment of the present invention.

### Detailed Description of the Embodiments

Reference will now be made in detail to exemplary embodiments, examples of which are illustrated in the accompanying drawings. When the following description refers to the accompanying drawings, the same numbers in different drawings represent the same or similar elements, unless otherwise represented. The implementations set forth in the following description of exemplary embodiments do not represent all implementations consistent with the present invention. Instead, they are merely examples of apparatuses and methods consistent with aspects related to the present invention as recited in the appended claims.

The terminology used in the present invention is for the purpose of describing particular embodiments only and is not intended to limit the present invention. As used in the present invention and the appended claims, the singular forms "a", "the", and "said" are intended to include the plural forms as well, unless the context clearly dictates otherwise. It should also be understood that the term "and/or" as used herein refers to and includes any and all possible combinations of one or more of the associated listed items. In addition, the term "at least one" as used herein represents any one of a plurality or any combination of at least two of a plurality.

It should be understood that although the terms first, second, third, etc. may be used in the present invention to describe various information, the information should not be limited by these terms. These terms are only configured to distinguish information of the same type from each other. For example, without departing from the scope of the present invention, first information may also be referred to as second information, and similarly, second information may also be referred to as first information. Depending on the context, the word "if" as used herein may be interpreted as "at" or "when" or "in response to determining".

In order to enable those skilled in the art to better understand the technical solutions in the embodiments of the present invention, and to make the above objects, features, and advantages of the embodiments of the present invention more apparent and easy to understand, the solutions in the embodiments of the present invention will be further described in detail below in conjunction with the accompanying drawings.

With the development of three-dimensional scanning technology, the three-dimensional scanning technology is widely applied in various fields such as medical treatment, industry, and archaeology to implement three-dimensional reconstruction on a target object. When a user uses a three-dimensional scanning device to scan the target object, in order to obtain an ideal reconstruction effect, generally, for different types of scanning environments, when images are collected using the three-dimensional scanning device, operational states of devices on the three-dimensional scanning device are different. Or there are also differences in processing methods of the images when three-dimensional reconstruction is subsequently performed using the images collected by the three-dimensional device.

For example, in order to adapt to different scanning environments, multiple operating modes can be configured for the three-dimensional scanning device. In the different operating modes, when the three-dimensional scanning device collects images, the suitable operational states can be configured for various components in the three-dimensional scanning device. Or when three-dimensional reconstruction is performed using the images collected by the three-dimensional device, the suitable processing methods can be selected, to achieve the best three-dimensional reconstruction effect.

At present, when switching the operating mode of the three-dimensional scanning device, it is mainly performed by manual control of the user. For example, a mode switching button can be set on the three-dimensional scanning device, and before scanning, the user can switch the operating mode of the three-dimensional scanning device to the suitable operating mode through the above mode switching button based on a type to which a current scanning environment belongs. However, in many scenarios, the user may forget to perform an operation of setting the operating mode, or the set operating mode is inaccurate, resulting in a poor effect of a finally reconstructed target object, and requiring re-performing three-dimensional reconstruction or even re-scanning, which seriously affects scanning efficiency and user experience.

For example, taking a three-dimensional scanning device applied to an oral scanning scenario as an example, when the user reconstructs a tooth model, it may be necessary to scan real teeth in an oral cavity, or it may be necessary to scan the tooth model outside the oral cavity. Since there are large differences between an intraoral environment and an extraoral environment, for example, in the intraoral environment, there are interferences such as gums, tongues, and buccal soft tissues, while the extraoral environment does not have the above problems, or compared with the extraoral environment, the brightness in the intraoral environment is lower, and so on. Thus, for the intraoral environment and the extraoral environment, there are differences in the processing methods of the images in a subsequent process of reconstructing teeth using the images. Therefore, it is necessary to set the different operating modes for the above two usage scenarios, and configure the suitable processing methods for each of the operating modes. However, when the user uses the device, the user usually forgets to switch the operating mode, resulting in a poor effect of a finally reconstructed three-dimensional tooth model, and requiring re-performing three-dimensional reconstruction or re-scanning.

Based on this, the embodiments of the present invention provide a control method for a three-dimensional scanning device, where a current scanning environment type of the three-dimensional scanning device can be automatically determined based on an image collected by the three-dimensional scanning device, and then an operating mode of the three-dimensional scanning device is automatically switched to a target operating mode adapted to the scanning environment type, or the user is prompted to switch the operating mode of the three-dimensional scanning device to the target operating mode adapted to the scanning environment type, thereby avoiding the problem that the user forgets to set the operating mode, resulting in a poor effect of a finally reconstructed three-dimensional model, and requiring re-performing three-dimensional reconstruction or re-scanning.

The control method for the three-dimensional scanning device provided by the embodiments of the present invention can be executed by the three-dimensional scanning device, or can be executed by a terminal device connected to the three-dimensional scanning device in a wireless or wired manner, where the terminal device can be a tablet, a laptop, etc. ; or some steps are executed by the three-dimensional scanning device, and some steps are executed by the terminal device.

For example, in some scenarios, when the three-dimensional scanning device itself has sufficient computing power, it can collect the images and perform three-dimensional reconstruction using the collected images to obtain a three-dimensional reconstruction model. Therefore, the three-dimensional scanning device can switch the operational states of the devices therein based on a scanning environment type, and switch the processing methods when performing three-dimensional reconstruction on the images.

In some scenarios, if the three-dimensional scanning device itself has limited computing power, it is necessary to leverage the terminal device to implement three-dimensional reconstruction. For example, scanning software installed on the terminal device can execute real-time three-dimensional reconstruction based on the images collected by the three-dimensional scanning device, and visualize a reconstruction result to the user. Accordingly, the three-dimensional scanning device can switch the operational states of its internal components based on a scanning environment type, and the scanning software switches the processing method when performing three-dimensional reconstruction on the images.

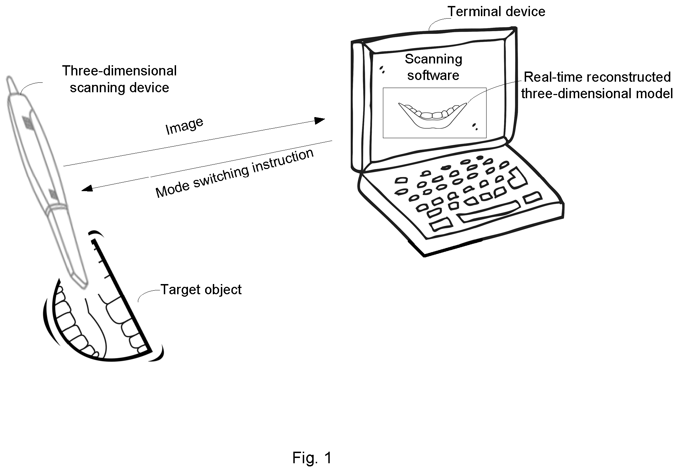

For example, as shown in Fig. 1, which is a schematic diagram of an application scenario according to the embodiments of the present invention, considering that the three-dimensional scanning device has limited computing power, images collected by the three-dimensional scanning device are usually sent to a terminal device with better processing performance connected in communication with the three-dimensional scanning device. Scanning software can be installed on the terminal device, and the scanning software performs real-time three-dimensional reconstruction based on the collected images and displays a reconstruction result to the user. At the same time, the scanning software can perform detection of a scanning environment type based on the images collected by the three-dimensional scanning device, and send a mode switching instruction to the three-dimensional scanning device based on a detection result to control the three-dimensional scanning device to perform mode switching. For example, controlling the three-dimensional scanning device to switch operational states of each component, and simultaneously, the three-dimensional scanning software can switch its own processing method when performing three-dimensional reconstruction on the images.

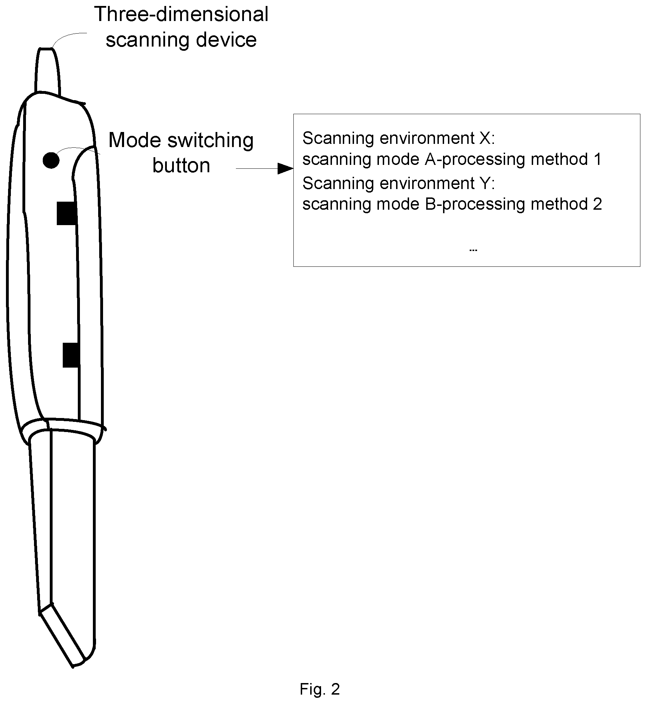

In order to adapt to different types of scanning environments, as shown in Fig. 2, multiple operating modes can be configured for the three-dimensional scanning device based on actual usage scenarios thereof, where each of the operating modes corresponds to one scanning environment type. For the different operating modes, operational state of each component, in the three-dimensional scanning device when collecting the images using the three-dimensional scanning device, and/or processing method of image processing when performing three-dimensional reconstruction using the images collected by the three-dimensional scanning device can be preset. In some embodiments of the present invention, the operating mode of the three-dimensional scanning device can include the operational state of each component in the three-dimensional scanning device and the processing method of three-dimensional scanning software matching the three-dimensional scanning device.

For example, for different scanning environments, in order to obtain the best reconstruction effect, during the three-dimensional reconstruction process, the processing methods applicable thereto are different. The processing method includes processing steps included in processing the image, processing algorithms used, processing parameters used during processing, and so on. The processing methods under the different operating modes can be pre-configured, and when switching to a certain operating mode, the processing method corresponding to the operating mode can be automatically retrieved subsequently to process the images to complete three-dimensional reconstruction.

For another example, for different scanning environments, when collecting the images, in order to obtain the images of better quality, the operational states of the components on the three-dimensional scanning device are also different. The operational state of the component can be operation parameters or states of various devices in the three-dimensional scanning device, for example, an on/off state of a fill light in the three-dimensional scanning device, or a brightness parameter of a fill light, or an on/off state of an anti-fog module, or an intensity of projected structured light, and so on.

A mode switching button can be provided on a three-dimensional scanning device, as shown in Fig. 2, to facilitate switching between the different operating modes, or the user can also switch the operating mode of the three-dimensional scanning device through scanning software.

In addition, an operating mode indicator light may be provided on the three-dimensional scanning device or the terminal device to indicate the current operating mode of the scanner.

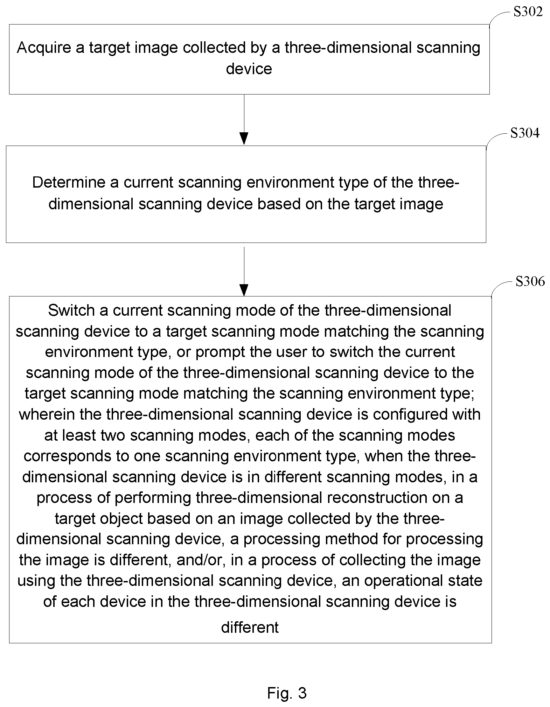

As shown in Fig. 3, which is a flowchart of the control method for a three-dimensional scanning device, the following steps can be included.

At step S302, a target image collected by the three-dimensional scanning device is acquired.

In the S302, the target image collected by the three-dimensional scanning device can be acquired, where the target image is configured to detect a current scanning environment type of the three-dimensional scanning device. In order to improve the accuracy of a detection result, the target image can be at least one frame of the images screened from the images collected by the three-dimensional scanning device that are suitable for performing scanning environment detection.

The three-dimensional scanning device can be an oral scanner, a facial scanner, an industrial scanner, a professional scanner, and can be configured to three-dimensional reconstruction of items such as teeth, human faces, human bodies, industrial products, industrial equipment, cultural relics, artworks, prostheses, medical instruments, buildings, etc.

At step S304, the current scanning environment type of the three-dimensional scanning device is determined based on the target image.

In the S304, the current scanning environment type of the three-dimensional scanning device can be determined based on the target image, for example, a neural network model or a deep neural network model can be configured to detect the target image. For example, a model for performing scanning environment type detection can be pre-trained, and then a trained model is configured to perform detection of the scanning environment type, or other methods can also be configured to detect the target image to determine the current scanning environment type of the three-dimensional scanning device.

At step S306, a current operating mode of the three-dimensional scanning device is switched to a target operating mode matching the scanning environment type, or the user is prompted to switch the current operating mode of the three-dimensional scanning device to the target operating mode matching the scanning environment type.

In the S306, after determining the current scanning environment type of the three-dimensional scanning device, the current operating mode of the three-dimensional scanning device can be automatically switched to the target operating mode matching the scanning environment type. For example, a correspondence relationship between scanning environment types and operating modes has been pre-configured, so the target operating mode can be directly determined based on the correspondence relationship, and the three-dimensional scanning device is controlled to switch to the target operating mode. For example, scanning software can send a mode switching instruction to the three-dimensional scanning device to control the three-dimensional scanning device to automatically switch to the target mode. In some scenarios, after determining the target operating mode matching the current scanning environment type, a prompt message can also be sent to the user, prompting the user to switch the current operating mode of the three-dimensional scanning device to the target operating mode. For example, the user can be prompted to switch the current operating mode to the target operating mode through voice prompts, text prompts, pop-up prompts, ringtone prompts, vibration prompts, and other methods.

After switching the three-dimensional scanning device to the target operating mode, each component in the three-dimensional scanning device can operate based on an operational state corresponding to the target operating mode, and/or when performing three-dimensional reconstruction using the images collected by the three-dimensional scanning device, a processing method corresponding to the target operating mode can be used to process the images to reconstruct a three-dimensional model of a target object.

Through the solution provided by the embodiments of the present invention, a scanning environment type of the three-dimensional scanning device can be automatically detected, and an operating mode matching the current scanning environment type can be automatically determined, and then the user is prompted to switch to the operating mode, or it is automatically switched to the operating mode, thereby avoiding the problem that the user forgets to set an operating mode, resulting in a mismatch between the operating mode and the scanning environment type, and a poor effect of the reconstructed three-dimensional model.

When scanning the target object for three-dimensional reconstruction, there are two scenarios: a first scenario is that there are fewer interfering objects around the target object, and there are fewer impurities in the images collected in the scanning process, mainly the target object, and in the first scenario, three-dimensional reconstruction can be directly performed using the collected images. A second scenario is that there are many interfering objects around the target object, and there are many impurities in the images collected in the scanning process. When the collected images are directly used for subsequent three-dimensional reconstruction, due to the interference of impurities, the accuracy and effect of the reconstructed three-dimensional model will be compromised. Therefore, in the second scenario, during the three-dimensional reconstruction process, data in the image that is irrelevant to three-dimensional reconstruction of the target object is first identified and subsequently marked. Subsequently, when performing three-dimensional reconstruction, this part of data does not need to participate in three-dimensional reconstruction, so as to improve the accuracy of the reconstructed three-dimensional model. Therefore, in some embodiments of the present invention, the different processing methods can refer to whether processing steps of processing the image includes a target step, where the target step is configured to determine data in the image that is irrelevant to a target object to be reconstructed, so as not to use these irrelevant data for three-dimensional reconstruction when subsequently generating a three-dimensional reconstruction model of the target object.

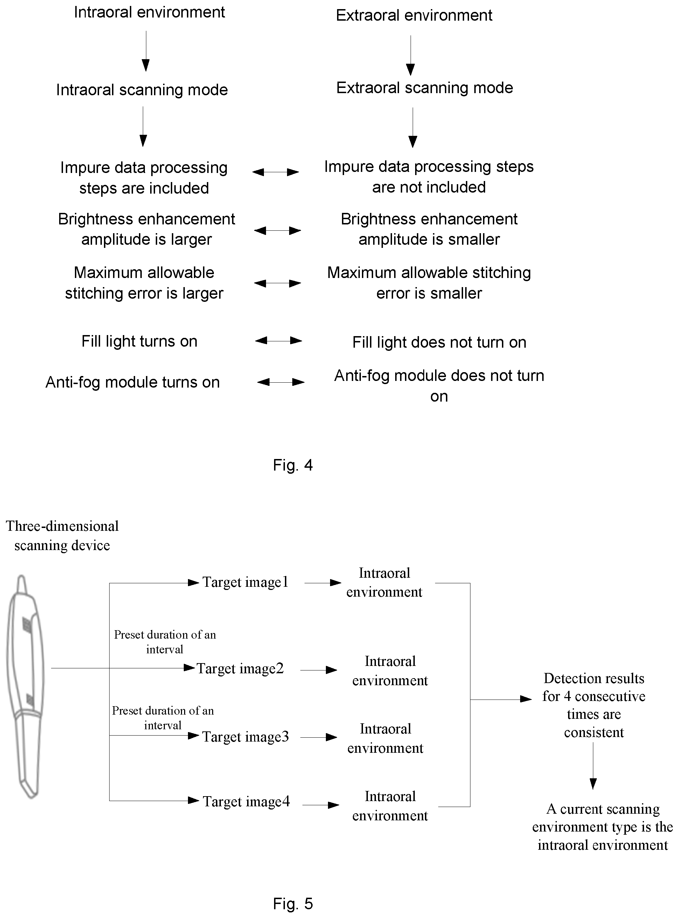

For example, in some embodiments of the present invention, the three-dimensional scanning device is an oral three-dimensional scanning device. When the oral three-dimensional scanning device scans teeth to reconstruct a three-dimensional model of the teeth, there are the following two scenarios: one is scanning real teeth of the user in an oral cavity. In this case, since the oral cavity of the user includes many interferences around the teeth such as tongues, buccal soft tissues, gums, etc., the collected images include many impurities. The other is scanning a tooth model outside the oral cavity, for example, scanning a tooth model made of wax, metal, resin, etc. In this case, since there is no interference around the teeth, there are fewer impurities in the collected images. Therefore, as shown in Fig. 4, scanning environment types can be divided into an intraoral environment and an extraoral environment, and operating modes can include an intraoral operating mode corresponding to the intraoral environment and an extraoral operating mode corresponding to the extraoral environment. When the operating mode is the intraoral operating mode, the processing steps of processing the images collected by the three-dimensional scanning device during the three-dimensional reconstruction process include the target step. When the operating mode is the extraoral operating mode, the processing steps of processing the images collected by the three-dimensional scanning device during the three-dimensional reconstruction process do not include the above target step. Data irrelevant to three-dimensional reconstruction of the target object can refer to regions such as tongues, buccal soft tissues, and parts of gums in the image. Image data corresponding to these regions in the image can be deleted first, and then a subsequent three-dimensional reconstruction processing flow is performed, which can avoid interference of these data on three-dimensional reconstruction.

In some embodiments of the present invention, the different processing methods to process the images collected by the three-dimensional scanning device can mean different processing parameters when processing the images collected by the three-dimensional scanning device, wherein the processing parameters can include at least one of the following: a brightness enhancement amplitude when performing brightness enhancement on the image, and a maximum allowable stitching error when stitching the images. For example, in the process of performing three-dimensional reconstruction using the images collected by the three-dimensional scanning device, considering that the environmental brightness in some scenarios is dim, a brightness of the image can be enhanced to improve the display effect of the reconstructed three-dimensional model. For different scenarios, however, the brightness of a scanning environment is also different, therefore, when performing brightness enhancement on an image, different brightness enhancement amplitudes can be applied to different scanning environment types to adjust the brightness of the image to a suitable brightness.

In addition, in the process of performing three-dimensional reconstruction using the images collected by the three-dimensional scanning device, it is necessary to stitch multiple frames of the images continuously collected by the three-dimensional scanning device to obtain a complete target object. For different scanning environments, in order to achieve successful stitching of the images, there are also certain differences in the maximum allowable stitching error when stitching the images. For example, in some scenarios, the target object is a rigid object, and its shape and position do not change during the scanning process. In this case, in order to ensure accurate stitching, the stitching error can be set to be smaller. In some scenarios, however, the target object is not a rigid object, and during the scanning process, the shape and position of the target object may change slightly. In this case, in order to ensure that the same region can be successfully stitched, the stitching error can be set to be larger. Therefore, the different maximum allowable stitching errors can be set based on characteristics of the scanned target object in different scanning environment types.

In some embodiments of the present invention, as shown in Fig. 4, the three-dimensional scanning device is an intraoral three-dimensional scanning device, the scanning environment type includes an intraoral environment and an extraoral environment, and the operating mode includes an intraoral operating mode corresponding to the intraoral environment, and an extraoral operating mode corresponding to the extraoral environment. Considering that in an intraoral scanning scenario, since the environmental brightness is often low, when performing brightness enhancement on the images, its enhancement amplitude should be set to be larger, while in an extraoral scanning scenario, since the environmental brightness is high, when performing brightness enhancement on the images, its enhancement amplitude should be set to be smaller. Therefore, when the operating mode is the intraoral operating mode, the brightness enhancement amplitude is set to a first amplitude, and when the operating mode is the extraoral operating mode, the brightness enhancement amplitude is set to a second amplitude, wherein the first amplitude is greater than the second amplitude. By setting different brightness enhancement amplitudes for different scanning environment types, it is ensured that the brightness of a processed image is adjusted to a suitable value, guaranteeing the display effect of the processed image.

In addition, considering that in the intraoral scanning scenario, since there are non-rigid objects such as gums and buccal sides, i.e., the shape and position of teeth may change slightly, which may affect the fluidity and accuracy of the images during a stitching process. The extraoral scanning scenario, however, does not have the above problems. Therefore, in the intraoral scanning scenario, the maximum allowable stitching error when stitching the images is set to be larger to ensure that the images can be successfully stitched. Thus, in the extraoral scanning scenario, the maximum allowable stitching error when stitching the images is set to be smaller to ensure stitching accuracy. Therefore, as shown in Fig. 4, in some embodiments of the present invention, when the operating mode is the intraoral operating mode, the maximum stitching error is set to a first stitching error, and when the operating mode is the extraoral operating mode, the maximum stitching error is set to a second stitching error, wherein the first stitching error is greater than the second stitching error. By setting the different stitching errors for different scanning environment types, it is ensured that the images can be successfully and accurately stitched in different scanning scenarios.

Furthermore, the different operating modes of the three-dimensional scanning device can mean the different operational states of components in the three-dimensional scanning device. For example, when the three-dimensional scanning instrument of the three-dimensional scanning device is an oral scanner, the different operational states of the components include whether a fill light module operates, whether an anti-fog module is turned on. For example: when the operating mode is the intraoral operating mode, the anti-fog module and the fill light module are turned on, and when the operating mode is the extraoral operating mode, the anti-fog module and the fill light module are turned off. The anti-fog module can include a heating element configured to prevent a lens of the three-dimensional scanning device from fogging. The fill light module can include a fill light configured to provide fill light for a collection range of the three-dimensional scanning device.

When the three-dimensional scanning device is a handheld scanner, the different operational states of the components include: whether a laser speckle module operates, whether a line laser module operates, whether a fringe light module operates, or whether a phase-shift fringe module operates, whether a visible light module operates, whether an invisible light module operates. For example: when the operating mode is a human figure operating mode, the visible light module is turned off, and the invisible light module is turned on, and when the operating mode is an industrial product operating mode, the visible light module is turned on, and the invisible light module is turned off.

For example: considering that differences also exist even within intraoral scanning scenarios. For instance, elderly patients may suffer from significant tooth loss, whereas others may possess intact dentition. For scenarios with different tooth loss situations, in the process of image processing to implement three-dimensional reconstruction, the processing methods of the images can also be adaptively adjusted to obtain more accurate three-dimensional reconstruction results. Therefore, in some embodiments of the present invention, the intraoral operating mode can be further subdivided into at least two sub-modes, wherein in different sub-modes, tooth loss situations in a scanned oral cavity are different, and the processing methods of image processing in different sub-modes are different. For example, the intraoral operating mode can be subdivided into an edentulous mode and a dentate mode, and the processing methods can be adaptively adjusted for these two modes respectively.

In an embodiment, in the edentulous mode and the dentate mode, determination criteria regarding data irrelevant to the three-dimensional reconstruction of the target object (i.e., teeth) are adjusted accordingly. For example, for buccal and lingual side data, in the dentate mode, all buccal and lingual side data will be treated as the above irrelevant data, while in the edentulous mode, lingual side data and more outer buccal side data will be treated as the above irrelevant data, but a part of the inner buccal side data and more dental arch data and gum data will be treated as data relevant to three-dimensional reconstruction, used for subsequent three-dimensional reconstruction. Moreover, when the edentulous mode is for a medical scenario involving full dentures, more parts of the buccal side or gum data can be selected to be retained for subsequent three-dimensional reconstruction.

Similarly, for the extraoral scanning scenario, it is similar. Since the extraoral scanning scenario is mainly for scanning tooth models, and the tooth models often include various materials, such as plaster, wax rims, metal, resin, etc. When performing three-dimensional reconstruction on the tooth models of different materials, the processing methods of the images can also be adaptively adjusted to obtain the more accurate three-dimensional reconstruction results. Therefore, in some embodiments of the present invention, the extraoral operating mode can be further subdivided into multiple sub-modes, wherein in different sub-modes, materials of the scanned tooth models are different, and the processing methods of image processing in different sub-modes are different. For example, when scanning a metal tooth model, the brightness of structured light projected by the three-dimensional scanning device can be enhanced, and during subsequent three-dimensional reconstruction, data of more small blocks can be retained, making the reconstructed three-dimensional model more complete.

In some embodiments of the present invention, in order to ensure the accuracy of a detection result of a current scanning environment type of the three-dimensional scanning device, the target image can be multiple frames of images. Each of the frames of target images in the multiple frames of the target images can be detected respectively to determine the current scanning environment type of the three-dimensional scanning device, obtaining the detection result corresponding to that frame of the target image. When detection results of the multiple frames of the target images are all consistent, then the detection results of the multiple frames of the target images are taken as the current scanning environment type of the three-dimensional scanning device. For example, as shown in Fig. 5, a detection count threshold can be set, assuming the detection count threshold is 4 times, then 4 frames of the target images can be acquired respectively from the images collected by the three-dimensional scanning device, and a current scanning environment type is determined based on each of the frames of the target images. When detection results for 4 consecutive times are all consistent, for example, all are intraoral environments, then the current detection result (intraoral environment) can be taken as the current scanning environment type of the three-dimensional scanning device. By determining the final detection result based on the detection results of the plurality of frames of the images, the accuracy of the detection result can be improved.

In some embodiments of the present invention, considering that the images continuously collected by the three-dimensional scanning device may all be images of the same region in a scanning environment, i.e., image content is roughly the same. When the multiple frames of the target images correspond to the same region, then detection results of determining a current scanning environment type of the three-dimensional scanning device based on the multiple frames of the target images will inevitably be consistent too, which is not helpful for improving the accuracy of detection results. Therefore, when acquiring the target image, one frame can be collected every preset duration, thereby ensuring that a collection time interval between adjacent two frames of the target images is long, i.e., adjacent two frames of the target images correspond to different regions in the scanning environment. In this way, the accuracy of the detection results determined based on the multiple frames of the target images is higher.

In some embodiments of the present invention, when performing detection on a current scanning environment type of the three-dimensional scanning device, considering that not all images collected by the three-dimensional scanning device are suitable for performing detection of the scanning environment types. For example, taking a scenario of scanning an oral cavity of a patient as an example, after the user clicks a "start scanning" button, they may not put an oral three-dimensional scanning device into the oral cavity of the patient for scanning in time, i.e., at this time, the images collected by the oral three-dimensional scanning device are not the images of a real scanning scenario, i.e., invalid images, and thus the results of using such images to detect the scanning environment types are also unreliable. Therefore, in order to improve detection efficiency, the above target images used for performing the scanning environment type detection can be valid images screened from the images collected by the three-dimensional scanning device, wherein the valid images are images that can be successfully stitched with other images to reconstruct a three-dimensional model of the target object during the three-dimensional reconstruction process. In some embodiments of the present invention, in order to improve the screening efficiency of the valid images, in the process of performing three-dimensional reconstruction using the images collected by the three-dimensional scanning device, upon determining that an image has successfully completed stitching with a preceding frame of the image and successfully implemented three-dimensional reconstruction, the collection time of the image is recorded, and the images whose collection time is after that of the image and that have successfully implemented three-dimensional reconstruction are then all determined as the valid images.

In some embodiments of the present invention, the timing of performing detection on a current scanning environment type of the three-dimensional scanning device based on the target images occurs when it is detected that the user triggers a designated interaction component, wherein after the designated interaction component is triggered, the images collected by the three-dimensional scanning device start to be used for three-dimensional reconstruction. For example, usually when the user starts scanning the target object, they can click a "start scanning" button on scanning software, at which point the scanning software starts acquiring the images collected by the three-dimensional scanning device and starts executing the three-dimensional reconstruction flow. Therefore, when it is detected that the user clicks the button, detection of the current scanning environment type can be performed.

In some embodiments of the present invention, the timing of performing detection on a current scanning environment type of the three-dimensional scanning device based on the target images occurs when it is detected that a difference between two frames of the images collected before and after by the three-dimensional scanning device is relatively large. Considering that in the scanning process, the user may have a case where a scanning environment switches, for example, after the user clicks a "start scanning" button on scanning software, they first scan the oral cavity of the patient, and after the scanning is complete, they move the three-dimensional scanning device out of the oral cavity of the patient to scan a tooth model outside the oral cavity. In order to be able to determine whether a scanning environment has changed and whether a step of re-performing scanning environment type detection is needed, two frames of the images continuously collected by the three-dimensional scanning device can be continuously compared. When it is determined that a difference in image content between two consecutive frames of the images exceeds a preset difference threshold, the step of determining the current scanning environment type of the three-dimensional scanning device based on the target images is performed.

In some embodiments of the present invention, before prompting the user to switch a current operating mode of the three-dimensional scanning device to a target operating mode matching the scanning environment type, it can be determined first whether the current operating mode of the three-dimensional scanning device is consistent with the target operating mode. When the current operating mode of the three-dimensional scanning device is not consistent with the target operating mode, the user is prompted to switch the current operating mode of the three-dimensional scanning device to the target operating mode matching the scanning environment type.

In some embodiments of the present invention, the method can be executed by a terminal device in communication connection with the three-dimensional scanning device, and the three-dimensional scanning device is configured to send a collected image to the terminal device, so that the terminal device performs three-dimensional reconstruction on the target object based on a received image, wherein the terminal device includes a display interface configured to display a real-time reconstructed three-dimensional model. When prompting the user to switch the current operating mode of the three-dimensional scanning device to the target operating mode matching a current scanning environment type, prompt information can be displayed through the display interface to prompt the user to switch the current operating mode of the three-dimensional scanning device to the target operating mode. For example, scanning software can be installed on the terminal device, and the scanning software usually displays a real-time reconstructed three-dimensional model to the user through an interaction interface, so that the user understands the current scanning situation. Therefore, the user can be prompted through text, patterns, a combination of text and patterns, etc. in the interaction interface, to prompt the user to switch the current operating mode to the target operating mode.

In some embodiments of the present invention, the three-dimensional scanning device can be the oral three-dimensional scanning device, scanning environment types include an intraoral environment and an extraoral environment. When determining the current scanning environment type, the target images can be input into a pre-trained scanning environment detection model, and the scanning environment detection model predicts the current scanning environment type. The scanning environment detection model can be trained through a large number of sample images labeled with the scanning environment types.

In some embodiments of the present invention, considering that extraoral environments are usually diverse, for example, in a scenario of scanning a tooth model extraorally, since materials of tooth models usually include many categories, such as metal, resin, wax, etc., and the sample images are difficult to cover all extraoral environment scenarios. Thus, a detection model trained using the sample images is difficult to accurately predict some scenarios not covered by the sample images, resulting in suboptimal accuracy of detection results of the pre-trained scanning environment detection model. In order to improve the performance of the scanning environment detection model, in some embodiments of the present invention, when training the above model, a contrastive learning mechanism can be introduced, i.e., when determining a loss of the above model, a contrastive loss can be introduced. By introducing the contrastive loss, the above model can be constrained, so that when the above model performs feature extraction on the images, for the images of the same category (i.e., images with the same scanning environment type), the extracted features are brought closer together as much as possible, and conversely, for the images of different categories, the extracted features are pushed as far apart as possible.

For example, in some embodiments of the present invention, when training a scanning environment detection model, at least two frames of the sample images can be acquired, wherein scanning environment types corresponding to the at least two frames of the sample images are the same. Then, a preset initial model can be configured to perform feature extraction on the at least two frames of the sample images respectively, obtaining the respective features of the at least two frames of the sample images. Then, based on a difference between a similarity of features of pairs of sample images among the at least two frames of the sample images and a preset similarity threshold, a target loss can be determined, and then model parameters of the initial model are adjusted based on the target loss to train the initial model, obtaining the above scanning environment detection model. Usually, when two frames of the sample images correspond to the same scanning environment type, then the features of the two frames of the sample images should also be similar. Therefore, a similarity threshold can be preset. When scanning environment types of two frames of the sample images are the same, then the similarity of the features of two frames of the sample images should be very close to the similarity threshold. Therefore, the target loss can be determined based on the closeness of the two, to train the initial model, so that for a trained model, when performing feature extraction on the images, for the images with the same scanning environment type, the extracted features are also closer.

In some embodiments of the present invention, when training the above scanning environment detection model, sample image triplets can also be acquired, where each of the sample image triplets includes a first sample image, a second sample image whose scanning environment type is the same as the scanning environment type of the first sample image, and a third sample image whose scanning environment type is different from the scanning environment type of the first sample image. Then, a preset initial model can be configured to perform feature extraction on the first sample image, the second sample image, and the third sample image respectively, obtaining their respective features. Then, based on a similarity of features of the second sample image and the first sample image, and a similarity of features of the third sample image and the first sample image, a target loss can be determined, and network parameters of the initial model are adjusted based on the target loss to train the initial model. A similarity of features of two frames of the sample images with the same scanning environment type should be higher than a similarity of features of two frames of the sample images with different scanning environment types. Based on such principle, the target loss is determined to adjust model parameters of the initial model, which can make the trained scanning environment detection model perform more accurate feature extraction on the images, and thus detection results are also more accurate.

In some embodiments of the present invention, considering that intraoral environments are often relatively similar, with little difference, thus for the images collected in the intraoral environment, image features thereof are often relatively similar, i.e., feature vectors of the images will be distributed within a rough range in a feature space. Therefore, for detection of the intraoral environments, a large number of sample images collected by the oral three-dimensional scanning device in the intraoral environment can also be acquired in advance, then feature extraction is performed on the sample images to obtain the features of the sample images, and a clustering center of features of the sample images is determined. For example, the feature vectors characterizing the features of the sample images can be obtained, and a clustering center of the feature vectors is determined. Subsequently, when determining a current scanning environment type of the oral three-dimensional scanning device based on the target images, feature extraction can be performed on the target images to obtain features of each frame of the target images, and then based on the closeness of the features of each frame of the target images to the feature clustering center, it is determined whether the current scanning environment type of the oral three-dimensional scanning device is an intraoral environment. For example, when the features of each frame of the target images are very close to the feature clustering center, it indicates that the scanning environment type corresponding to the target images is the intraoral environment, wherein the closeness can be represented by a distance between the features of each frame of the target images and the feature clustering center, for example, it can be represented by Euclidean distance, Manhattan distance, etc. When the distance between the features of each frame of the target images and the feature clustering center is less than a preset distance threshold, it is considered that the features of each frame of the target images and the feature clustering center are close, i.e., it is determined that the scanning environment type corresponding to the target images is the intraoral environment.

It is not difficult to understand that the solutions described in the above embodiments can be freely combined to obtain new solutions in the absence of conflict, which are not listed one by one in the embodiments of the present invention due to space limitations.

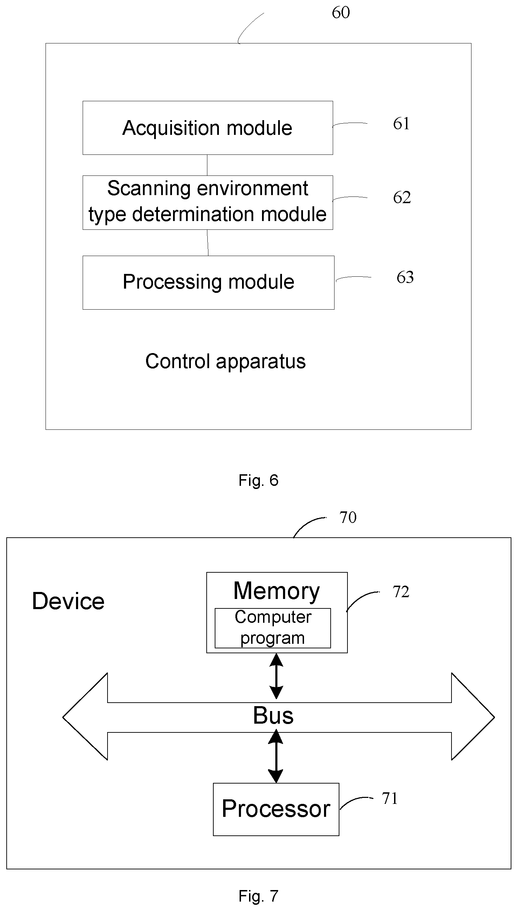

Correspondingly, the embodiments of the present invention further provide a control apparatus for the three-dimensional scanning device. As shown in Fig. 6, the control apparatus includes an acquisition module 61, a scanning environment type determination module 62, and a processing module 63.

The acquisition module 61 is configured to acquire a target image collected by the three-dimensional scanning device;

The scanning environment type determination module 62 is configured to determine a current scanning environment type of the three-dimensional scanning device based on the target image.

The processing module 63 is configured to switch a current operating mode of the three-dimensional scanning device to a target operating mode matching the scanning environment type, or prompt the user to switch the current operating mode of the three-dimensional scanning device to the target operating mode matching the scanning environment type.

The three-dimensional scanning device is configured with at least two operating modes, each of the operating modes corresponds to one scanning environment type, when the three-dimensional scanning device is in the different operating modes, in the process of performing three-dimensional reconstruction on a target object based on the image collected by the three-dimensional scanning device, a processing method for processing the image is different; and/or, in the process of collecting the image using the three-dimensional scanning device, an operational state of each device in the three-dimensional scanning device is different.

For steps of the above control apparatus executing the control method for the three-dimensional scanning device, reference can be made to the description in the above method embodiments, and details are not repeated here.

Further, the embodiments of the present invention further provide a terminal device. As shown in Fig. 7, the terminal device includes a processor 71, a memory 72, and computer instructions stored in the memory 72 and executable by the processor 71. The processor 71, when executing the computer instructions, implements the control method for the three-dimensional scanning device in any one of the above embodiments.

For steps of the above terminal device executing the control method for the three-dimensional scanning device, reference can be made to the description in the above method embodiments, and details are not repeated here.

Further, the embodiments of the present invention further provide a control system for the three-dimensional scanning device is provided. The control system includes the terminal device mentioned in the above embodiments, and the three-dimensional scanning device in communication connection with the terminal device. The three-dimensional scanning device is configured to collect an image of an area to be scanned and send the image to the terminal device.

The embodiments of the present invention further provide a computer-readable storage medium, on which a computer program is stored. The program, when executed by a processor, implements the control method for the three-dimensional scanning device of any one of the foregoing embodiments.

Computer-readable media include permanent and non-permanent, removable and non-removable media, and information storage can be implemented by any method or technology. Information can be computer-readable instructions, data structures, program modules, or other data. Examples of computer storage media include, but are not limited to, Phase-change Random Memory (PRAM), Static Random Access Memory (SRAM), Dynamic Random Access Memory (DRAM), other types of Random Access Memory (RAM), Read-Only Memory (ROM), Electrically Erasable Programmable Read-Only Memory (EEPROM), flash memory or other memory technology, Compact Disc Read-Only Memory (CD-ROM), Digital Versatile Disc (DVD) or other optical storage, magnetic cassette tape storage, magnetic disk storage or other magnetic storage devices, or any other non-transmission medium, which can be configured to store information that can be accessed by a computing device. As defined herein, the computer-readable media does not include transitory media, such as modulated data signals and carrier waves.

From the description of the above embodiments, it can be clearly understood by skilled in the art that the embodiments of the present invention can be implemented by means of software plus a necessary general hardware platform. Based on such understanding, the solutions of the embodiments of the present invention, in essence or the part contributing to the related art, can be embodied in the form of a software product. The computer software product can be stored in a storage medium, such as ROM/RAM, magnetic disk, optical disc, etc., including several instructions to cause a computer device (which can be a personal computer, a server, or a network device, etc. ) to execute the methods of the various embodiments of the present invention or certain parts of the embodiments.

The systems, apparatuses, modules, or units described in the above embodiments can be implemented by computer chips or entities, or by products having certain functions. A typical implementation device is a computer, and the form of the computer can be a personal computer, a laptop computer, a cellular phone, a camera phone, a smartphone, a personal digital assistant, a media player, a navigation device, an email sending and receiving device, a game console, a tablet computer, a wearable device, or a combination of any of these devices.

The various embodiments in the specification are described in a progressive manner, and the same or similar parts between the various embodiments can be referred to each other, with each of the embodiments focusing on differences from other embodiments. In particular, for the apparatus embodiments, since they are basically similar to the method embodiments, the description is relatively simple, and for relevant parts, reference can be made to the description of the section of the method embodiments. The apparatus embodiments described above are merely illustrative, and the modules described as separate components may or may not be physically separate, and when implementing the solutions of the embodiments of the present invention, the functions of each module can be implemented in the same or a plurality of software and/or hardware. Some or all of the modules can also be selected according to actual needs to achieve the purpose of the solution of this embodiment. Those of ordinary skill in the art can understand and implement them without creative effort.

The above are only implementations of the embodiments of the present invention. It should be pointed out that for those of ordinary skill in the art, several improvements and modifications may be made without departing from the principles of the embodiments of the present invention, and these improvements and modifications should also be regarded as the protection scope of the embodiments of the present invention.

### Industrial Applicability

The solutions provided by the present invention can be applied to the field of three-dimensional scanning technology. The solutions of the present invention can automatically determine a current scanning environment type of a three-dimensional scanning device based on an image collected by the three-dimensional scanning device, and then automatically switch an operating mode of the three-dimensional scanning device to a target operating mode adapted to the scanning environment type, or prompt the user to switch the operating mode of the three-dimensional scanning device to the target operating mode adapted to the scanning environment type, thereby avoiding the problem that the user forgets to set the operating mode, resulting in a poor effect of a finally reconstructed three-dimensional model, and requiring re-performing three-dimensional reconstruction or re-scanning.

## Claims

1. A control method for a three-dimensional scanning device, comprising:
acquiring a target image collected by the three-dimensional scanning device;
determining a current scanning environment type of the three-dimensional scanning device based on the target image;
switching a current operating mode of the three-dimensional scanning device to a target operating mode matching the scanning environment type, or prompting a user to switch the current operating mode of the three-dimensional scanning device to the target operating mode matching the scanning environment type;
wherein the three-dimensional scanning device is configured with at least two operating modes, each of the operating modes corresponds to one scanning environment type, when the three-dimensional scanning device is in the different operating modes, in a process of performing three-dimensional reconstruction on a target object based on an image collected by the three-dimensional scanning device, a processing method for processing the image is different, and/or, an operational state of each component in the three-dimensional scanning device during a process of collecting the image using the three-dimensional scanning device is different.

2. The method as claimed in claim 1, wherein the different processing method comprises: whether a processing step of processing the image comprises a target step; wherein the target step is configured to determine data in the image that is irrelevant to three-dimensional reconstruction of a target object to be reconstructed, to ignore the data when generating a three-dimensional model of the target object.

3. The method as claimed in claim 2, wherein the three-dimensional scanning device is an intraoral three-dimensional scanning device, scanning environment types comprise an intraoral environment and an extraoral environment, and the operating modes comprise an intraoral operating mode corresponding to the intraoral environment, and an extraoral operating mode corresponding to the extraoral environment;
when the operating mode is the intraoral operating mode, the processing steps for processing the image comprises the target step;
when the operating mode is the extraoral operating mode, the processing step of processing the image does not comprise the target step.

4. The method as claimed in claim 1, wherein the different processing method comprises: processing parameters when processing the image are different, the processing parameters comprising at least one of the following:
an enhancement amplitude when performing brightness enhancement on the image, and a maximum allowable stitching error when stitching the image.

5. The method as claimed in claim 4, wherein the three-dimensional scanning device is an intraoral three-dimensional scanning device, scanning environment types comprise an intraoral environment and an extraoral environment, and the operating modes comprise the intraoral operating mode corresponding to the intraoral environment, and the extraoral operating mode corresponding to the extraoral environment; wherein
when the operating mode is the intraoral operating mode, the enhancement amplitude is a first amplitude, and the maximum stitching error is a first stitching error;
when the operating mode is the extraoral operating mode, the enhancement amplitude is a second amplitude, and the maximum stitching error is a second stitching error;
wherein the first amplitude is greater than the second amplitude, and the first stitching error is greater than the second stitching error.

6. The method as claimed in claim 3 or 5, wherein the intraoral operating mode comprises at least two sub-modes, a shedding situation of teeth in a scanned oral cavity is different under different sub-modes, and the processing method for processing the image is different under the different sub-modes; and/or
the extraoral operating mode comprises multiple sub-modes, a material of a scanned tooth model is different under different sub-modes, and the processing method for processing the image is different under the different sub-modes.

7. The method as claimed in claim 1, wherein the target image comprises multiple frames, a collection time interval between two adjacent frames of the target images in the multiple frames of the target images exceeds a preset duration, and the determining the current scanning environment type of the three-dimensional scanning device based on the target image comprises:
detecting the current scanning environment type of the three-dimensional scanning device respectively based on each frame of the target image in the multiple frames of the target images, to obtain a detection result;
in response to the detection results corresponding to the multiple frames of the target images being consistent, taking a current detection result as the current scanning environment type of the three-dimensional scanning device; and/or
the target image is a valid image screened from images collected by the three-dimensional scanning device, and the valid image is obtained based on the following manner: after three-dimensional reconstruction is successfully implemented using images collected by the three-dimensional scanning device, in response to the current frame image being successfully stitched with a previous frame image of the current frame image, recording a collection time of the current frame image; and determining an image, which has a collection time later than that the collection time of the current frame image and for which three-dimensional reconstruction is successfully implemented, as a valid image.

8. The method as claimed in claim 1, wherein the determining the current scanning environment type of the three-dimensional scanning device based on the target image comprises:
in a case where it is detected that the user triggers a designated interaction component, determining the current scanning environment type of the three-dimensional scanning device based on the target image, wherein after the designated interaction component is triggered, images collected by the three-dimensional scanning device start to be used for three-dimensional reconstruction; or
in response to determining that a difference in scene content of two consecutive frames of the images collected by the three-dimensional scanning device exceeds a preset difference threshold, determining the current scanning environment type of the three-dimensional scanning device based on the target image.

9. The method as claimed in claim 1, wherein the prompting the user to switch the current operating mode of the three-dimensional scanning device to the target operating mode matching the scanning environment type comprises:
determining whether the current operating mode of the three-dimensional scanning device is consistent with the target operating mode;
in response to the current operating mode of the three-dimensional scanning device being inconsistent with the target operating mode, prompting the user to switch the current operating mode of the three-dimensional scanning device to the target operating mode matching the scanning environment type.

10. The method as claimed in claim 9, wherein the method is executed by a terminal device in communication connection with the three-dimensional scanning device, and the three-dimensional scanning device is configured to send the collected image to the terminal device, to enable the terminal device perform the three-dimensional reconstruction on the target object based on the received image; the terminal device comprises a display interface which is configured to display a real-time reconstructed three-dimensional model;
the prompting the user to switch the current operating mode of the three-dimensional scanning device to the target operating mode matching the scanning environment type comprises:
displaying prompt information through the display interface to prompt the user to switch the current operating mode of the three-dimensional scanning device to the target operating mode matching the scanning environment type.

11. The method as claimed in claim 1, wherein the three-dimensional scanning device comprises an intraoral three-dimensional scanning device, scanning environment types comprise an intraoral environment and an extraoral environment, determining the scanning environment type by detecting the target image through a pre-trained scanning environment detection model, and the scanning environment detection model is trained and obtained by the following manner:
acquiring at least two frames of sample images, wherein scanning environment types corresponding to the at least two frames of the sample images are the same; performing feature extraction on the at least two frames of the sample images respectively using a preset initial model to obtain respective features of the at least two frames of the sample images;
determining a target loss based on a difference between a similarity of features of pairs of sample images among the at least two frames of the sample images and a preset similarity threshold, and adjusting model parameters of the initial model based on the target loss to train and obtain the scanning environment detection model; or
acquiring sample image triplets, wherein each of the sample image triplets comprises a first sample image, a second sample image having a scanning environment type same as the scanning environment type of the first sample image, and a third sample image having a scanning environment type different from the scanning environment type of the first sample image; performing feature extraction on the first sample image, the second sample image, and the third sample image respectively using the preset initial model to obtain respective features; and determining a target loss based on a similarity of features of the second sample image with features of the first sample image, and a similarity of features of the third sample image with features of the first sample image, and adjusting the model parameters of the initial model based on the target loss to train and obtain the scanning environment detection model.

12. The method as claimed in claim 1, wherein the three-dimensional scanning device comprises an intraoral three-dimensional scanning device, the scanning environment type comprises an intraoral environment and an extraoral environment, and the determining the current scanning environment type of the three-dimensional scanning device based on the target image comprises:
performing feature extraction on the target image to obtain features of the target image; and
determining whether the current scanning environment type of the intraoral three-dimensional scanning device is the intraoral environment based on a closeness degree of the features of the target image to a preset feature clustering center; wherein the feature clustering center is a clustering center of features of multiple frames of sample images, and the sample images are images collected by the intraoral three-dimensional scanning device in the intraoral environment.

13. A control apparatus for a three-dimensional scanning device, comprising:
an acquisition module, configured to acquire a target image collected by the three-dimensional scanning device;
a scanning environment type determination module, configured to determine a current scanning environment type of the three-dimensional scanning device based on the target image;
a processing module, configured to switch a current operating mode of the three-dimensional scanning device to a target operating mode matching the scanning environment type, or prompting a user to switch the current operating mode of the three-dimensional scanning device to the target operating mode matching the scanning environment type; wherein
the three-dimensional scanning device is configured with at least two operating modes, each of the operating modes corresponds to one scanning environment type, when the three-dimensional scanning device is in the different operating modes, in a process of performing three-dimensional reconstruction on a target object based on an image collected by the three-dimensional scanning device, a processing method for processing the image is different, and/or, an operational state of each component in the three-dimensional scanning device during a process of collecting the image using the three-dimensional scanning device is different.

14. A terminal device, comprising: a processor, a memory, and computer instructions stored in the memory and executable by the processor, wherein the processor implements, when executing the computer instructions, the method according to any one of claims 1 to 12.

15. A control system for a three-dimensional scanning device, comprising: a terminal device according to claim 14, and the three-dimensional scanning device in communication connection with the terminal device, wherein the three-dimensional scanning device is set to collect an image of an area to be scanned and send the image to the terminal device.

16. A computer-readable storage medium, wherein a computer program is stored on the computer-readable storage medium, and the computer program implements, when executed by a processor, the method according to any one of claims 1 to 12.
